# EUROPEAN PATENT SPECIFICATION

(11) **EP 3 473 295 B1**
(45) Date of publication and mention of the grant of the patent: **20.01.2021**
(21) Application number: 17824504.9
(22) Date of filing: 04.07.2017
(51) Int. Cl.: A61N 1/06, A61N 1/05, A61N 1/32, A61H 39/08, A61B 18/14

(54) **HIGH-FREQUENCY HAND PIECE FOR SKIN TREATMENT**
HOCHFREQUENZHANDSTÜCK ZUR HAUTBEHANDLUNG
PIÈCE À MAIN HAUTE FRÉQUENCE POUR TRAITEMENT DE LA PEAU

(30) Priority: 04.07.2016 KR 20160084225
(43) Date of publication of application: 24.04.2019
(73) Proprietor: Panace Co., Ltd., Seongnam-si, Gyeonggi-do 13215 (KR)
(72) Inventor: YOON, Sung Tae, Seongnam-si Gyeonggi-do 13622 (KR)
(74) Representative: Manitz Finsterwald Patent- und Rechtsanwaltspartnerschaft mbB
(86) International application number: PCT/KR2017/007102
(87) International publication number: WO 2018/008948

(56) References cited:
- WO-A2-2007/064718
- KR-A- 20160 038 126
- KR-B1- 100 946 363
- KR-B1- 101 122 526
- KR-B1- 101 188 710
- KR-B1- 101 363 000
- KR-B1- 101 363 000
- KR-B1- 101 369 271
- US-A1- 2016 114 181

## Description

### BACKGROUND

### 1. Field of the Invention

The present disclosure relates to a high frequency handpiece for skin treatment, and more particularly, to a high frequency handpiece for skin treatment, which directly transmits a high frequency current to the dermal layer between the epidermal layer and the subcutaneous fat layer of the skin to activate the cell tissue, thus enabling elastic skin to be maintained and skin aging to be minimized.

### 2. Description of the Related Art

Skin covers our entire body and is formed of three layers including the epidermal layer, the dermal layer, and the subcutaneous fat layer. The epidermal layer is the outermost layer of the skin, which is divided into several layers including cornified layer, clear layer, granular layer, spinous layer, and basal layer according to their locations and functions, and provides function of protection, defense, secretion, etc. The dermal layer is located below the epidermal layer and adjacent to the base layer, constitutes the majority of the skin, and is divided into: papillary dermis composed of water, proteins, saccharides, mucopolysaccharides, minerals, and inorganic salts in gel-like state, which has capillary vessels associated with blood circulation and lymphatic vessels carrying lymph; and reticular dermis composed of collagen which is collagenous fiber associated with the wrinkles of the skin, elastin which is resilient fiber that provides elasticity to the skin, and ground substrate (reservoir of water). Finally, the subcutaneous fat layer is located between the dermis, muscles and bones, contains a large amount of fat, and forms the lowest layer of the skin. The subcutaneous fat layer spreads evenly throughout the human body, providing elasticity-maintaining and buffering action of absorbing external pressure and impact, thereby not only protecting the inside of the body against damages, but also preventing the loss of body heat and maintaining body temperature.

In order to prevent the aging of the skin described above, besides the traditional method of massaging skin with a cosmetic material containing nutrients, a method of injecting a serum into skin using a syringe or the like is widely used. However, this skin treatment method has disadvantage that it is not only costly, but also can have many side effects depending on the physical constitution of the treated person, in which case it may rather adversely affect the skin.

Additionally, a method of indirectly delivering high frequency or the like from the epidermal layer to the dermal layer of the skin is also used, but this method has a shortcoming in that high frequency is indirectly transmitted to the dermal layer through the epidermal layer, thus providing less satisfactory effect compared to the massage-type skin treatment method, and also causing inconvenience and high cost burden by requiring treatment to be continued periodically.

High frequency skin treatment method, which transmits high frequency currents to activate cell tissue directly to the dermal layer of the skin through the use of needle, thus maintaining skin elasticity and minimizing aging of skin, is receiving increasing attention. An example can be found in Korean Patent Laid-Open No. 10-2015-0007938, entitled "Skin treatment apparatus". The related art disclosed in Korean Patent Laid-Open No. 10-2015-0007938 includes a treatment unit which is inserted into a patient's skin to stimulate the skin, a holder to which the treatment unit is removably attached and which transmits a signal to the treatment unit, and a handpiece to which a holder part is mounted and which supplies a signal to the holder. The treatment unit includes a treatment needle inserted into the skin; a support plate connected to the treatment needle and restricted from moving by the skin; and an insertion plate connected to the support plate and mounted on the holder. To prevent risks such as the transmission of germs, used treatment unit is discarded and replaced with a new one. The replacement of the treatment unit with a new treatment unit after every use involves a process of gripping and separating a small-sized treatment unit with tweezers, and then replacing with a new one, which is a very inconvenient and difficult task, and there is also a high risk of secondary infection when the treatment unit is lost.

Other examples of state of the art treatment methods and devices for skin treatment can be found in US-A-2016/114181, KR-B-10-1363000, WO-A-2007/064718 and KR-B-10-1188710.

### SUMMARY

The present disclosure is designed to solve the problems described above, and it is a further object of the present invention to provide a high frequency handpiece for skin treatment, in which a cartridge with a needle for insertion into the skin is easily separated from and coupled to a handle, thus solving difficulty related with the replacement of the needle, and enabling more hygienic replacement, and also preventing a risk of accident due to misplaced needle. This object is solved by the invention according to claim 1.

The object of the present invention described above can thus be achieved by a high frequency handpiece for skin treatment, which may include: a handle of which one end is connected to a high frequency generator and the other end has an opening formed thereon, in which the handle has a cylindrical shape to allow grip by hand; and a needle cartridge removably coupled to one side of the handle and including a needle formed thereon to output a high frequency to skin when supplied with power, in which the needle cartridge includes a holder having both open sides and a passage formed therein; a needle assembly coupled to one side of the holder and having the needle formed therein to be inserted into the skin; a connector coupled to an opening on the other side of the holder, connected to one side of the needle assembly, and having a fitting part formed at the other side; and a power plug removably fitted into the fitting part of the connector and is coupled to the handle to supply power. The high frequency handpiece skin treatment is characterized in that the connector has a shape of a circular rod, formed of a conductive metal material, and includes the fitting part formed at one end thereof with a plurality of cuts arranged in a circumferential direction. Furthermore, it includes a mounting surface formed on the other side by vertically cutting along an outer circumferential surface for close contact with the needle.

The holder may include a coupling part including a needle insertion hole formed at one side for receiving the needle assembly to be inserted therein, and a through hole formed at the other side for receiving a connector to be inserted therein, and the coupling part may include a fitting slot in a ' 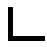 ' shape on an outer circumferential surface thereof, for coupling with a fastening portion of the handle.

The fastening part of the handle includes a fastening hole formed at one end, into which the fastening part is inserted, and a stopper formed on an inner circumferential surface of the fastening hole to be inserted into the fitting slot.

According to the present disclosure, because the cartridge having needle integrally formed therein can be easily separated from the handle to be replaced with a new one, difficulty arising from the replacement of the needle can be solved, and hygienic replacement of the needle can be provided, which prevents the risk of secondary infection, and also prevents loss of a fine needle and undesirable accident that may be caused by the misplaced needle.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
Fig. 1 is a perspective view showing a high frequency handpiece for skin treatment according to an embodiment of the present disclosure;
Fig. 2 is an exploded perspective view showing a high frequency handpiece for skin treatment according to an embodiment of the present disclosure;
Fig. 3 is a partially enlarged perspective view of Fig. 2; and
Figs. 4 and 5 are partial cross-sectional views showing an interior of a high frequency handpiece for skin treatment according to an embodiment of the present disclosure.

### DETAILED DESCRIPTION

According to the present invention, a high frequency handpiece for skin treatment may include: a handle of which one end is connected to a high frequency generator and the other end has an opening formed thereon, in which the handle has a cylindrical shape to allow grip by hand; and a needle cartridge fitted to one side of the handle and including a needle formed thereon to output a high frequency to skin when supplied with power, in which the needle cartridge includes a holder having both open sides and a passage formed therein; a needle assembly coupled to one side of the holder and having the needle formed therein to be inserted into the skin; a connector coupled to an opening on the other side of the holder, connected to one side of the needle assembly, and having a fitting part formed at the other side; and a power plug removably fitted into the fitting part of the connector and is coupled to the handle to supply power. The high frequency handpiece skin treatment is characterized in that the connector has a shape of a circular rod, formed of a conductive metal material, and includes the fitting part formed at one end thereof with a plurality of cuts arranged in a circumferential direction. Furthermore, it includes a mounting surface formed on the other side by vertically cutting along an outer circumferential surface for close contact with the needle.

Hereinafter, preferred embodiments of the present disclosure will be described in detail with reference to the accompanying drawings. It should be noted that the same elements are denoted by the same reference numerals as possible in the accompanying drawings. Further, the detailed description of well-known functions and configurations that may obscure the gist of the present disclosure will be omitted.

In addition, it should be understood that the terms used in the description and the appended claims which will be described below should not be construed as limited to general and dictionary meanings, but interpreted based on the meanings and concepts corresponding to technical aspects of the present disclosure on the basis of the principle that the inventor is allowed to define terms appropriately for the best explanation.

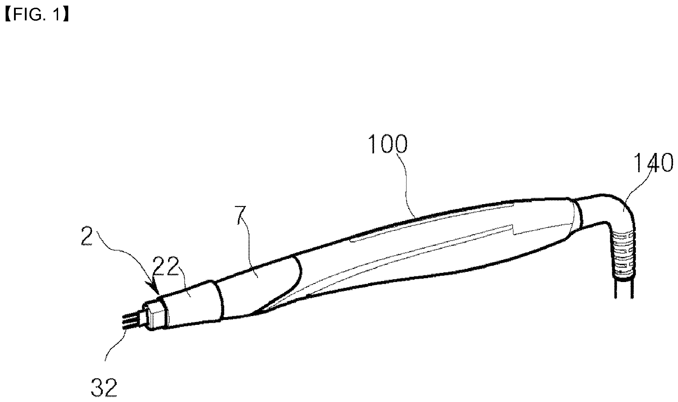

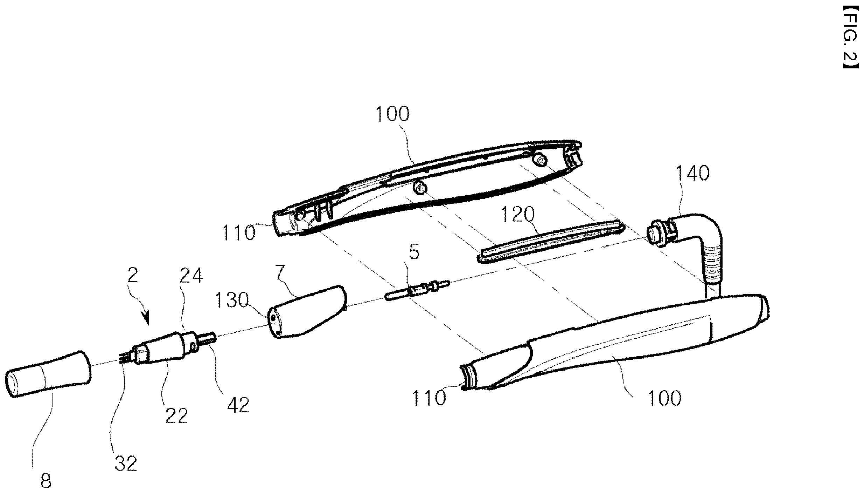

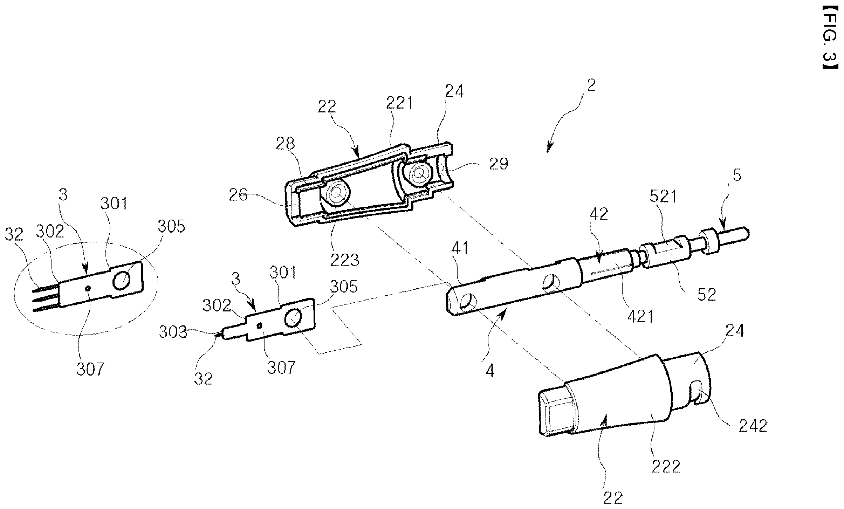

In the accompanying drawings, Fig. 1 is a perspective view showing a high frequency handpiece for skin treatment according to an embodiment of the present disclosure, Fig. 2 is an exploded perspective view showing a high frequency handpiece for skin treatment according to an embodiment of the present disclosure, Fig. 3 is a partially enlarged perspective view of Fig. 2, and Figs. 4 and 5 are partial cross-sectional views showing an interior of a high frequency handpiece for skin treatment according to an embodiment of the present disclosure.

The high frequency handpiece for skin treatment according to an embodiment of the present disclosure includes a handle 100 having a cylindrical shape to allow grip by hand, of which one end is connected to a high frequency generator (not illustrated), and the other end has an opening formed therein; and a needle cartridge 2 fitted into one side of the handle 100 and including a needle 32 for outputting high frequency to the skin when supplied with power.

The handle 100 has a predetermined length and has a cylindrical shape, while having a streamlined shape to provide good grip feeling and to prevent slippage. In addition, the handle 100 may be divided into two parts, and house therein parts such as a conductor 120 and a power plug 140 for supplying power to inside.

A cap 7 is coupled to a front end of the handle 100 for fitting with the coupling part 24 of the needle cartridge 2. A stopper 130 is formed on an inner circumferential surface of the cap 7 to be inserted into a fitting slot 242.

The needle cartridge 2 is removable, that is, the needle cartridge 2 is attachable to and detachable from the handle 100 and thus can be discarded and replaced with a new one after every use.

The needle cartridge 2 includes: a holder 22 having both open sides and a passage formed therein; a needle assembly 3 coupled to one side of the holder 22 and having the needle 32 therein to be inserted into the skin; a connector 4 coupled to the other opening of the holder 22 and connected to one side of the needle assembly 3, and having a fitting part 42 formed on the other side; and a power plug 140 removably fitted into the fitting part 42 of the connector 4 and coupled to the handle 100 to supply power.

The holder 22 includes a needle insertion hole 26 formed at one side to receive the needle assembly 3 to be inserted thereinto, and the coupling part 24 formed at the other side, having a through hole 29 to receive the connector 4 to be inserted thereinto.

The holder 22 is formed as a cylindrical body that is formed by coupling two sub-bodies, i.e., first and second bodies 221 and 222 to face each other, and has a tapered shape such that the diameter increases from a front end where the needle insertion hole 26 is formed, toward a rear end where the coupling part 24 is formed, thus allowing easy grip with the thumb and index finger.

The coupling part 24 is formed in a cylindrical shape at the rear end of the holder 22, and is formed in a smaller diameter than the rear end of the holder 22, and has symmetrical fitting slots 242 recessed into a concave shape that is a ' 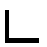 ' shape on the outer circumferential surface of the coupling part 314 to receive the coupling part 110 of the handle 100 to be fitted therein.

The stopper 130 is formed on an inner circumferential surface of the cap 7 to be inserted into the fitting slots 242.

Spaces are formed inside the first and second bodies 221 and 222, and guide steps 28 are formed on a cutting plane of the first and second bodies 221 and 222, along edges of the spaces.

The guide steps 28 are configured for a close contact with an outer periphery of the needle assembly 3 to be described below. Because the needle assembly 3 can be brought into close contact with the guide steps 28, the needle assembly 3 can be stably secured.

The needle assembly 3 is a thin metal plate having conductivity, and includes a through hole 29 formed at one side to be coupled to the connector 4, and the sharp-pointed needle 32 formed at the other end.

The needle assembly 3 is formed such that width thereof decreases by one or two levels starting from a portion opposite the needle 32, into a first step 301, a second step 302 and a third step 303.

The first to third steps 301 to 303 are shaped to fit the guide steps 28 of the holder 22.

The needle assembly 3 includes a through hole 305 formed at one side, through which a coupling shaft 223 that is passed through holder 22 and the connector 4 is passed, and a fixing hole 307 formed on the other side, into which a pin formed in the holder 22 is fitted.

Therefore, when the needle assembly 3 is coupled to the holder 22 and then coupled to the connector 4, the needle assembly 3 can be firmly secured so as not to be moved.

The needle 32 is sharply-pointed to be inserted into the skin, and is formed of a conductive material to receive a high frequency signal from the outside and transmit the same to the skin.

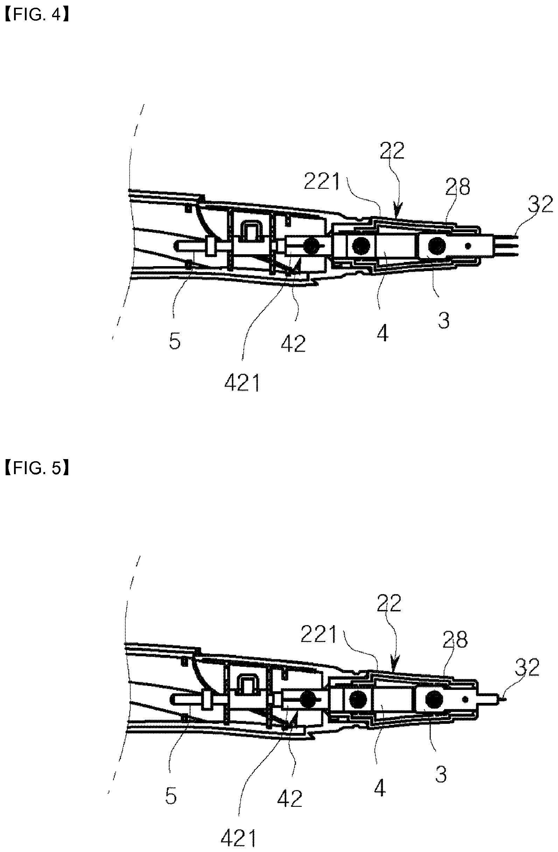

One, or two or more, that is, a plurality of needles 32 may be provided. Fig. 4 shows an example where there are three needles 32, and Fig. 5 shows an example where there is only one needle 32.

The connector 4 is a circular rod with conductivity, which has a mounting surface 41 formed at one side by vertically cutting the outer circumferential surface, and a plurality of cuts 421 arranged in a circumferential direction on the other side having hollow cylindrical shape, that is, on the fitting part 42.

The needle assembly 3 is brought into close contact with the mounting surface 41 of the connector 4.

The connector 4and the needle assembly 3 may be integrated as a screw is coupled through the mounting surface 41, with a portion of the screw being coupled to the needle assembly 3.

The power plug 140 is a circular rod with conductivity, of which one end is coupled to the fitting part 42 of the connector 4 by interference-fitting and the other end is connected to the conductor 120 by fitting.

The power plug 140 has an extended portion 52 in the middle portion where the outer diameter is increased, and a flat portion that forms a groove portion 521. The groove portion 521 is inserted into the conductor 120 to serve as a latching jaw, thus preventing the power plug 140 from falling off accidentally.

Hereinafter, the operation of the present disclosure with the configuration described above will be described.

The connector 4 is inserted into one sides of the first and second bodies 221 and 222 constituting the holder 22, and then the needle assembly 3 is inserted into the holder 22 on the other side. Then, the first and second bodies 221 and 222 are coupled to each other by screw-fastening, according to which both the needle assembly 3 and the connector 4 are also screw-fastened and integrated. As a result, the needle cartridge 2 is assembled.

The assembled needle cartridge 2 is fitted into the fastening part 110 of the handle 100. At this time, the cap 7 is engaged with a leading end of the handle 100 to improve the fixing force.

Meanwhile, since the needle 32 exposed to the outside can be dangerous, a lid 8 is coupled to one side of the holder 22 to protect the needle 32 in order to prevent an accident and damage to the needle 32.

When it is necessary to use the needle, the user removes the lid 8 and holds the handle 100 and allows the needle 32 to be inserted into the skin, and then applies a high frequency to perform the treatment.

After completing the treatment, the user rotates the holder 22 to cause the stopper 130 to be separated from the fitting slot 242, and then pulls the holder 22 outward to separate the needle cartridge 2 from the handle 100.

Therefore, the used needle cartridge 2 can be discarded and the new needle cartridge 2 can be coupled to the handle 100 to be used, thereby ensuring hygienic process and safety.

While the embodiments of the present disclosure have been described with reference to the accompanying drawings, those with ordinary knowledge in the technical field of the present disclosure will be able to understand that the present disclosure can be embodied into difference and more detailed modes, without departing from the technical concept or without modifying essential characteristics thereof. Accordingly, it will be understood that the exemplary embodiments described above are only illustrative, and should not be construed as limiting.

### [Industrial Applicability]

The present disclosure relates to a high frequency handpiece for skin treatment and can be used as a medical device for skin treatment.

The present invention is set out in the appended claims. The embodiments, examples or aspects according to the present description which do not fall within the scope of said claims are provided for illustrative purposes only and do not form part of the present invention.

## Claims

1. A high frequency handpiece for skin treatment, comprising:
a handle (100), of which one end is connected to a high frequency generator and the other end has an opening formed therein;
a needle cartridge (2) removably coupled to one side of the handle (100) and having a needle (32) formed therein to be inserted into skin and transmit a high frequency to the skin,
wherein the needle cartridge (2) comprises:
a holder (22) having both open sides and a passage formed therein;
a needle assembly (3) coupled to one side of the holder (22) and having the needle (32) formed therein;
a connector (4) coupled to an opening on the other side of the holder (22), connected to one side of the needle assembly (3), and having a fitting part (42) formed at the other side; and
a power plug (140) removably fitted into the fitting part (42) of the connector (4) and is coupled to the handle (100) to supply power,
**characterized in that** the connector (4) has a shape of a circular rod, formed of a conductive metal material, and includes the fitting part (42) formed at one end thereof with a plurality of cuts (421) arranged in a circumferential direction, and
a mounting surface (41) formed on the other side by vertically cutting along an outer circumferential surface for close contact with the needle (32).

2. The high frequency handpiece for skin treatment according to claim 1, wherein the holder (22) comprises a coupling part (24) and a needle insertion hole (26) formed at one side for receiving the needle assembly (3) to be inserted therein, and a through hole (29) formed at the other side for receiving a connector (4) to be inserted therein, and
the coupling part (24) includes a fitting slot (242) in a ' 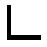 ' shape on an outer circumferential surface thereof, for coupling with a fastening portion (110) of the handle (100).

3. The high frequency handpiece for skin treatment according to claim 1, wherein the handle comprises
a cap (7) coupled to one side thereof to receive a coupling part (24) of the needle cartridge (2) to be fitted thereinto, and
a stopper (130) formed on an inner circumferential surface of the cap (7) to be inserted into a fitting slot (242).

4. The high frequency handpiece for skin treatment according to claim 1, wherein the needle assembly (3) is a thin conductive metal plate and includes through holes (305) formed at one side that is coupled to the connector (4), and one, or a plurality of sharp-pointed needles (32) at the other end.

## Patentansprüche

1. Hochfrequenzhandstück zur Hautbehandlung, umfassend:
einen Griff (100), von dem ein Ende mit einem Hochfrequenzgenerator verbunden ist und das andere Ende eine darin geformte Öffnung aufweist;
eine Nadelkartusche (2), die entfernbar mit einer Seite des Griffs (100) gekoppelt ist und eine Nadel (32) aufweist, die darin geformt ist, um in die Haut eingeführt zu werden und eine hohe Frequenz auf die Haut zu übertragen,
wobei die Nadelkartusche (2) umfasst:
einen Halter (22), der zwei offene Seiten und einen darin geformten Durchgang aufweist;
eine Nadelanordnung (3), die mit einer Seite des Halters (22) gekoppelt ist und in der die Nadel (32) geformt ist;
einen Verbinder (4), der mit einer Öffnung auf der anderen Seite des Halters (22) gekoppelt ist, mit einer Seite der Nadelanordnung (3) verbunden ist und ein auf der anderen Seite geformtes Passstück (42) aufweist;
und
einen Netzstecker (140), der entfernbar in das Passstück (42) des Verbinders (4) eingepasst ist und mit dem Griff (100) gekoppelt ist, um Strom zu liefern,
**dadurch gekennzeichnet, dass** der Verbinder (4) eine Form eines kreisförmigen Stabes hat, der aus einem leitenden Metallmaterial gebildet ist, und das an einem Ende davon gebildete Passstück (42) mit mehreren in Umfangsrichtung angeordneten Schnitten (421) umfasst; und
eine Montagefläche (41), die auf der anderen Seite durch vertikales Schneiden entlang einer äußeren Umfangsfläche zum engen Kontakt mit der Nadel (32) geformt ist.

2. Hochfrequenzhandstück zur Hautbehandlung nach Anspruch 1, wobei der Halter (22) ein Kopplungsteil (24) und ein Nadeleinführungsloch (26), die an einer Seite geformt sind, um die darin einzufügende Nadelanordnung (3) aufzunehmen, und ein Durchgangsloch (29) umfasst, das an der anderen Seite zur Aufnahme eines darin einzufügenden Verbinders (4) geformt ist, und
das Kopplungsteil (24) einen Passschlitz (242) in einer "L" -Form auf einer Außenumfangsfläche davon zum Koppeln mit einem Befestigungsabschnitt (110) des Griffs (100) umfasst.

3. Hochfrequenzhandstück zur Hautbehandlung nach Anspruch 1, wobei der Griff umfasst:
eine Kappe (7), die mit einer Seite davon gekoppelt ist, um ein Kopplungsteil (24) der Nadelkartusche (2), das darin eingepasst werden soll, aufzunehmen, und
eine Stoppeinrichtung (130), die auf einer inneren Umfangsfläche der Kappe (7) zum Einsetzen in einen Passschlitz (242) ausgebildet ist.

4. Hochfrequenzhandstück zur Hautbehandlung nach Anspruch 1, wobei die Nadelanordnung (3) eine dünne leitende Metallplatte ist und Durchgangslöcher (305), die an einer Seite ausgebildet sind, die mit dem Verbinder (4) gekoppelt ist, und eine oder mehrere scharfspitzige Nadeln (32) an dem anderen Ende aufweist.

## Revendications

1. Pièce à main à haute fréquence pour traitement de la peau, comprenant :
une poignée (100), dont une extrémité est connectée à un générateur de haute fréquence et dont l'autre extrémité comporte une ouverture formée à l'intérieur ;
un chariot à aiguille (2) couplé de manière amovible sur un côté de la poignée (100) et ayant une aiguille (32) formée à l'intérieur et destinée à être insérée dans la peau et à transmettre une haute fréquence à la peau, dans laquelle le chariot à aiguille (2) comprend :
un support (22) ayant deux côtés ouverts et un passage formé à l'intérieur ;
un assemblage à aiguille (3) couplé à un côté du support (22) et ayant l'aiguille (32) formée à l'intérieur ;
un connecteur (4) couplé à une ouverture sur l'autre côté du support (22), connecté à un côté de l'assemblage à aiguille (3), et ayant une partie de raccord (42) formée de l'autre côté ; et
une prise électrique (140) engagée de façon amovible dans la partie de raccord (42) du connecteur (4) et qui est couplée à la poignée (100) pour alimenter une puissance,
**caractérisé en ce que** le connecteur (4) a la forme d'une barre circulaire, formé d'un matériau métallique conducteur, et inclut la partie de raccord (42) formée à une extrémité de celle-ci avec une pluralité de découpes (421) agencées dans une direction circonférentielle, et
une surface de montage (41) formée sur l'autre côté par une découpe verticale le long d'une surface circonférentielle extérieure pour un contact intime avec l'aiguille (32).

2. Pièce à main à haute fréquence pour traitement de la peau selon la revendication 1, dans laquelle le support (22) comprend une partie de couplage (24) et un trou d'insertion pour aiguille (26) formé sur un côté afin de recevoir l'assemblage à aiguille (3) qu'il s'agit d'insérer dans celui-ci, et un trou traversant (29) formé sur l'autre côté pour recevoir un connecteur (4) qu'il s'agit d'insérer dans celui-ci, et
la partie de couplage (24) inclut une fente de raccord (242) sous une forme en "L" sur une surface circonférentielle extérieure de celle-ci, pour le couplage avec une portion de fixation (110) de la poignée (100).

3. Pièce à main à haute fréquence pour traitement de la peau selon la revendication 1, dans laquelle la poignée comprend :
un capuchon (7) couplé à un côté de celle-ci afin de recevoir une partie de couplage (24) du chariot à aiguille (2) destiné à être engagé dans celle-ci, et
un élément d'arrêt (130) formé sur une surface circonférentielle intérieure du capuchon (7) et destiné à être inséré dans une fente de raccord (242).

4. Pièce à main à haute fréquence pour traitement de la peau selon la revendication 1, dans laquelle l'assemblage à aiguille (3) est une plaque mince en métal conducteur et inclut des trous traversants (305) formés sur un côté qui est couplé au connecteur (4), et une aiguille ou une pluralité d'aiguilles à pointe acérée (32) à l'autre extrémité.
